# EUROPEAN PATENT APPLICATION

(11) **EP 0 693 297 A1**
(43) Date of publication of application: **24.01.1996**
(21) Application number: 95111272.1
(22) Date of filing: 18.07.1995
(51) Int. Cl.: A61M 1/34, A61M 1/16

(54) **Dialysis apparatus**

(30) Priority: 18.07.1994 IT BO940336
(71) Applicant: BELLCO S.p.A., I-41037 Mirandola(Modena) (IT)
(72) Inventor: Cianciavicchia, Domenico, I-64040 Cavuccio (IT); Morselli, Massimo, I-41038 S. Felice sul Panaro (IT)
(74) Representative: Jorio, Paolo

(57) **Abstract**

An apparatus hemodiafiltration (1) presenting a filter (6); a first tube (3) for feeding blood from an artery (4) to the filter (6); a second tube (8) for feeding blood from the filter (6) to a vein (9) communicating with the artery (4) via a fistula (10); and a machine (13) for preparing the dialysis solution whereby toxic substances are removed from the blood in the filter (6); the apparatus (1) also presenting means (18) for varying the quantity of saline ions in the blood fed along the second tube (8); means (16) for detecting the quantity of saline ions in the blood fed along the first tube (3); and means (21) for processing the above findings, to define a parameter indicating the amount of purified blood recirculated from the vein (9), via the fistula (10), to the artery (4), and therefore indicating the effectiveness of the treatment.

## Description

The present invention relates to a dialysis apparatus.

As is known, patients subjected to dialysis treatment present a fistula hydraulically connecting the artery from which the blood to be purified is drawn, to the vein into which the purified blood is fed; and one of the parameters best indicating the effectiveness of the treatment is the amount of purified blood fed from the vein, via the fistula, to the artery, and back into the external blood circuit.

It is an object of the present invention to provide a dialysis apparatus featuring a device for detecting the amount of purified blood recirculated into the external blood circuit.

It is a further object of the present invention to provide a method of determining the amount of purified blood recirculated into the external blood circuit.

According to the present invention, there is provided a dialysis apparatus comprising:
a filter assembly;
a first tube for feeding the blood to be purified from an artery of the patient to said filter assembly;
a second tube for feeding the purified blood from said filter assembly to a vein of the patient, said vein communicating hydraulically with said artery via a fistula; and
a dialysis machine for preparing the dialysis solution whereby the toxic substances in the blood are removed by said filter assembly;
characterized in that it comprises:
means for varying the percentage quantity of saline ions in the blood fed along the second tube;
first means for detecting, after said variation, the percentage quantity of saline ions in the blood fed along the second tube;
second means for detecting, after said variation, the percentage quantity of saline ions in the blood fed along the first tube; and
means for processing the above findings, to define a parameter indicating the amount of purified blood recirculated from said vein, via said fistula, to said artery, and therefore indicating the effectiveness of the dialysis treatment.

According to the present invention, there is also provided a method of determining the amount of purified blood recirculated into the external blood circuit in a dialysis apparatus comprising:
a filter assembly;
a first tube for feeding the blood to be purified from an artery of the patient to said filter assembly;
a second tube for feeding the purified blood from said filter assembly to a vein of the patient, said vein communicating hydraulically with said artery via a fistula; and
a dialysis machine for preparing the dialysis solution whereby the toxic substances in the blood are removed by said filter assembly;
characterized in that it comprises:
a first step in which means vary the percentage quantity of saline ions in the blood fed along said second tube;
a second step in which means detect the percentage quantity of saline ions in the blood fed along said second tube following said variation;
a third step in which means detect the percentage quantity of saline ions in the blood fed along said first tube following said variation; and
a fourth step in which means process the above findings; the outcome of said processing indicating the amount of purified blood recirculated from said vein, via said fistula, to said artery, and therefore indicating the effectiveness of the dialysis treatment.

A preferred, non-limiting embodiment of the present invention will be described by way of example with reference to the accompanying drawings, in which:
Figure 1 shows a block diagram of a dialysis apparatus;
Figure 2 shows an operating block diagram of the Figure 1 apparatus.

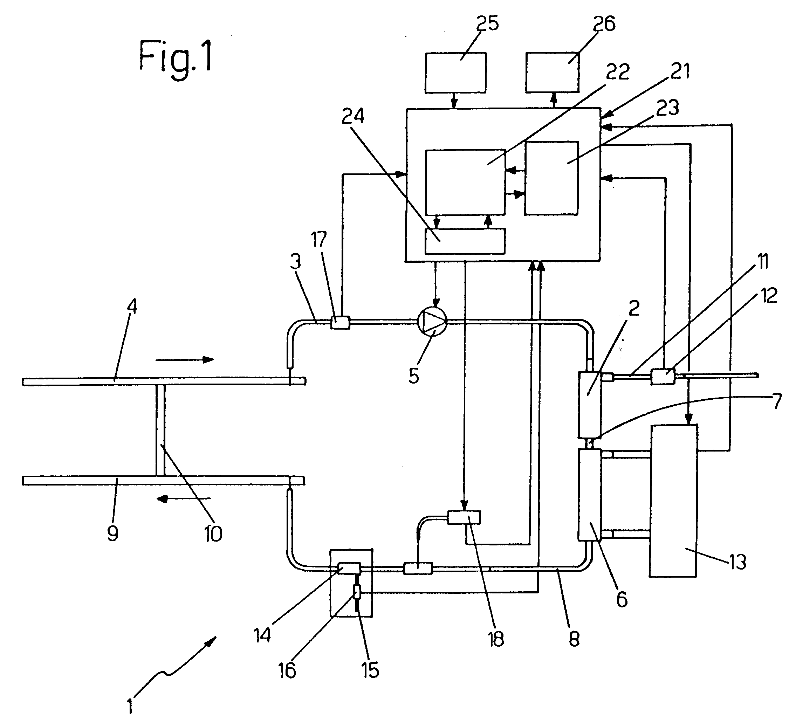

Number 1 in Figure 1 indicates a dialysis apparatus comprising:
a known haemofilter 2;
a tube 3 for feeding the blood to be purified from an artery 4 (shown schematically) of the patient to the inlet of haemofilter 2;
a preferably peristaltic pump 5 along tube 3;
a known haemodialyzer 6;
a tube 7 for feeding the blood from haemofilter 2 to the inlet of haemodialyzer 6;
a tube 8 for feeding the purified blood from haemodialyzer 6 to a vein 9 of the patient, said vein 9 communicating hydraulically with artery 4 via a fistula 10;
a tube 11 for feeding to a drain (not shown) the ultrafiltered fluid withdrawn from the blood by haemofilter 2;
a conductivity sensor 12 along tube 11;
a known dialysis machine 13 for preparing the dialysis solution whereby the toxic substances in the blood are removed by haemodialyzer 6;
a known haemofilter 14 along tube 8;
a tube 15 for feeding to a drain (not shown) the ultrafiltered fluid withdrawn from the blood by haemofilter 14;
a conductivity sensor 16 along tube 15;
a conductivity sensor 17 along tube 3;
a device 18 for infusing, upstream from haemofilter 14, saline ions into the blood fed along tube 8; and
an electronic control unit 21 for controlling pump 5, machine 13 and device 18, and to which sensors 12, 15 and 17 are connected.

Control unit 21 comprises a central data processing unit 22, a memory block 23, and a timing block 24, and is connected to a keyboard 25 and a display 26.

As is known, blood substantially comprises red cells, white cells, platelets, and plasma which in turn substantially comprises water, protein and saline ions; and a conductivity sensor generates an electric signal proportional to the electric conductivity of the fluid under examination. If the fluid being examined is blood, its electric conductivity is proportional to the percentage quantity of saline ions contained in it, so that a conductivity sensor installed along an external blood circuit generates an electric signal proportional to the percentage quantity of saline ions in the blood.

It is also known that the ultrafiltered fluid is perfectly equivalent to plasma with no protein, so that a conductivity sensor installed along the ultrafiltered fluid circuit generates an electric signal proportional to the percentage quantity of saline ions in the blood from which the ultrafiltered fluid is derived. In other words, sensor 17 determines directly the percentage quantity of saline ions in the blood fed along tube 3, sensor 12 determines indirectly the percentage quantity of saline ions in the blood fed along tube 3, and sensor 16 determines indirectly the percentage quantity of saline ions in the blood fed along tube 8.

The method of determining the amount of purified blood recirculated into the external blood circuit substantially comprises:
a first step to vary the percentage quantity of saline ions in the blood fed along tube 8;
a second step to detect the percentage quantity of saline ions in the blood fed along tube 8 following said variation;
a third step to detect the percentage quantity of saline ions in the blood fed along tube 3 following said variation; and
a fourth step in which the above findings are compared; the outcome of comparison indicating the amount of purified blood recirculated from vein 9 via fistula 10 to artery 4.

As already stated, the amount of purified blood recirculated constitutes a parameter whereby to evaluate the effectiveness of the treatment.

The percentage quantity of saline ions in the blood fed along tube 8 may be varied in several ways.

In a first solution of the first step, using a syringe, the operator injects a predetermined quantity of saline ions into tube 8; and, after infusion, the operator enables control unit 21 via keyboard 25, to determine by means of the sensors the percentage quantity of saline ions along the various tubes, and to process the incoming data to determine the amount of purified blood recirculated from vein 9 to artery 4.

In a second solution of the first step, once control unit 21 is programmed via keyboard 25, device 18 provides for feeding a predetermined quantity of saline ions into tube 8. As already stated, device 18 is controlled by unit 21, and may provide for a single infusion, or (with the aid of block 24) for a series of programmed timed infusions for continually controlling the amount of purified blood recirculated from vein 9 to artery 4. In this case, infusion is automatically enabled by control unit 21, and the relative data is processed after each infusion. By way of example, device 18 may be designed and operate in exactly the same way as a straightforward heparin pump.

A third solution of the first step provides for varying the operating characteristics of machine 13, e.g. the composition and supply of the dialysis solution, etc. In this case also, control unit 21, by controlling machine 13, provides for automatically varying the percentage of saline ions along tube 8, and for processing the relative data after each variation.

A fourth solution of the first step provides for inverting filter assembly 2, 6 for as long as required for the findings, which temporary alteration to the external blood circuit may be made either manually or by means of a device controlled by unit 21.

In any case, the processing results may be memorized in block 23 for retrieval prior to the next dialysis treatment, thus enabling recirculation of the purified blood and hence the effectiveness of the treatment to be controlled between one treatment and the next. The processing data and results may also be displayed on display 26.

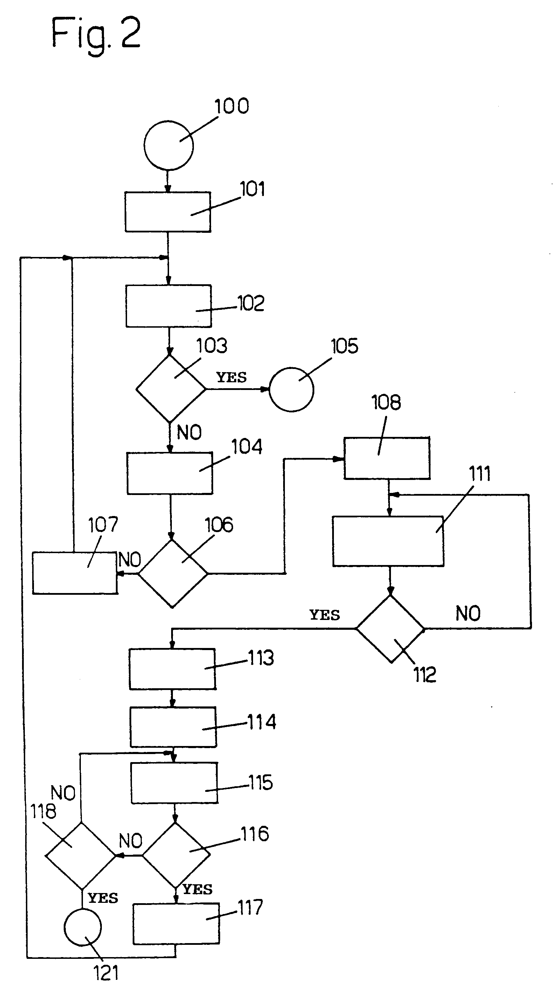

By way of example of the operation of apparatus 1, an operating cycle will now be described with reference to Figure 2. From a start block 100, a block 101 enables sensor 17 or 12 to determine the percentage quantity A of saline ions in the blood along tube 3, enables sensor 16 to determine the percentage quantity V of saline ions in the blood along tube 8, and provides for memorizing the detected value A in a first table in block 23, for memorizing the detected value V in a second table in block 23, and for initiating (t=0) timing block 24. Block 101 then goes on to block 102 which, after a predetermined sampling time tc (e.g. 15 seconds), enables sensor 17 or 12 to once more determine the percentage quantity A of saline ions in the blood along tube 3, and enables sensor 16 to determine the percentage quantity V of saline ions in the blood along tube 8. Block 102 then goes on to block 103 which determines whether the dialysis treatment is terminated, by comparing the time lapse (t) from initiation of block 24 with the dialysis treatment time (set by the operator via keyboard 25). In the event of a positive response, block 103 goes on to end-of-cycle block 105; conversely, block 103 goes on to block 104.

Block 104 compares the values A and V detected in block 102 with the values A and V memorized respectively in the first and second tables, and then goes on to block 106 which determines whether values A and V are stable, i.e. whether the outcome of the comparison between the values A and V detected in block 102 and the values A and V memorized in the first and second tables is below a respective predetermined threshold value As, Vs. In the event of a positive response, block 106 goes on to block 108; conversely, block 106 goes on to block 107; and both blocks 107 and 108 provide for deleting the values A and V currently memorized in the first and second tables, and for entering into the first and second tables the values A and V detected in block 102. Block 107 then goes back to block 102 to determine (after time lapse tc) further values A and V. The aim of the routine defined by blocks 102, 104, 106, 107 is to find values A and V (subsequently memorized in block 108) which may be considered stable in time. That is, each sampling time tc, values A and V are sampled until the difference between them and the previously memorized values A and V is below respective threshold values As and Vs.

Block 108 goes on to a block 111 wherein the operator is requested (via display 26) to enable measurement of the saline ion recirculation index R. Alternatively, a program may be memorized in block 23 for automatically enabling measurement of index R. Block 111 then goes on to block 112 which determines the response of the operator and/or the presence of said program in block 23. In the event the operator enables measurement of index R via keyboard 25, and/or the presence of said enabling program in block 23 is determined, block 112 goes on to block 113; conversely, block 112 goes back to block 111.

Block 113 provides, according to one of the solutions described above, for varying the percentage quantity of saline ions in the blood fed along tube 8, and takes the time t elapsed so far as the start time ts of the variation. Block 113 then goes on to block 114 which enables sensor 17 or 12 to determine the percentage quantity A of saline ions in the blood fed along tube 3, enables sensor 16 to determine the percentage quantity V of saline ions in the blood fed along tube 8, and provides for memorizing the detected value A in a third table in block 23, and for memorizing the detected value V in a fourth table in block 23. Block 114 then goes on to block 115 which, after a predetermined sampling time tc (e.g. 15 seconds), enables sensor 17 or 12 to again determine the percentage quantity A of saline ions in the blood fed along tube 3, enables sensor 16 to determine the percentage quantity V of saline ions in the blood fed along tube 8, and then goes on to block 116 which determines whether the values A and V determined in block 115 are below the values A and V memorized in the third and fourth tables. In the event of a positive response, block 116 goes on to block 117; conversely, block 116 goes on to block 118 which deletes the values A and V memorized in the third and fourth tables, memorizes in the third and fourth tables the values A and V determined in block 115, and then determines whether the difference between the time elapsed so far and the start time ts of the saline ion variation operation exceeds a given time value. In the event of a negative response, block 118 goes back to block 115; conversely, in the event of a positive response, block 118 goes on to block 121 which enables alarm signals (not shown) in that, even after a predetermined time lapse from the start of the saline ion variation operation, increasingly high values A and V have been determined, which may indicate a malfunction of apparatus 1. The routine defined by blocks 114, 115, 116, 118 is aimed at finding peak values A and V within a series of values A and V determined after each variation of the saline ions. That is, for each sampling time tc, values A and V are sampled until the peak values A and V are determined. In block 117, the values A and V memorized in the third and fourth tables are processed to define an index R indicating the amount of purified blood recirculated from vein 9 via fistula 10 to artery 4, and defined, for example, by the percentage ratio between peak value V and peak value A.

The advantages of the present invention will be clear from the foregoing description.

In particular, it provides for an apparatus which, on the basis of a set variation in the saline ions in the blood fed along tube 8, and on the basis of a number of findings, provides for determining the amount of purified blood recirculated, and so evaluating the effectiveness of the dialysis treatment.

The advantages of the present invention will be clear from the foregoing description.

In particular, it provides for an apparatus which, on the basis of a set variation in the saline ions in the blood fed along tube 8, and on the basis of a number of findings, provides for determining the amount of purified blood recirculated, and so evaluating the effectiveness of the dialysis treatment.

Clearly, changes may be made to apparatus 1 and the method as described and illustrated herein without, however, departing from the scope of the present invention.

In particular, sensor 16, like sensor 17, may be installed along tube 8 to directly determine the percentage quantity of saline ions in the blood fed along tube 8; apparatus 1 may present a further pump along tube 8; and sensors 12, 16, 17 may be either invasive or noninvasive.

## Claims

**1)** A dialysis apparatus comprising:
a filter assembly (2, 6);
a first tube (3) for feeding the blood to be purified from an artery (4) of the patient to said filter assembly (2, 6);
a second tube (8) for feeding the purified blood from said filter assembly (2, 6) to a vein (9) of the patient, said vein (9) communicating hydraulically with said artery (4) via a fistula (10); and
a dialysis machine (13) for preparing the dialysis solution whereby the toxic substances in the blood are removed by said filter assembly (2, 6);
characterized in that it comprises:
means (18) for varying the percentage quantity of saline ions in the blood fed along the second tube (8);
first means (16) for detecting, after said variation, the percentage quantity of saline ions in the blood fed along the second tube (8);
second means (17 or 12) for detecting, after said variation, the percentage quantity of saline ions in the blood fed along the first tube (3); and
means (21) for processing the above findings, to define a parameter indicating the amount of purified blood recirculated from said vein (9), via said fistula (10), to said artery (4), and therefore indicating the effectiveness of the dialysis treatment.

**2)** An apparatus as claimed in Claim 1, characterized in that said first (16) and second (17 or 12) detecting means comprise respective electric conductivity sensors.

**3)** An apparatus as claimed in Claim 2, characterized in that said second detecting means comprise a conductivity sensor (17) installed along said first tube (3).

**4)** An apparatus as claimed in Claim 2, characterized in that said filter assembly comprises a first haemofilter (2) from which extends a third tube (11) for channeling the ultrafiltered fluid; and said second detecting means comprise a conductivity sensor (12) installed along said third tube (11).

**5)** An apparatus as claimed in Claim 2, characterized in that said first detecting means comprise a conductivity sensor (16) installed along said second tube (8).

**6)** An apparatus as claimed in Claim 2, characterized in that it comprises a second haemofilter (14) installed along said second tube (8) and from which extends a fourth tube (15) for channeling the ultrafiltered fluid; and said first detecting means comprise a conductivity sensor (16) installed along said fourth tube (15).

**7)** An apparatus as claimed in any one of the foregoing Claims, characterized in that said means (18) for varying the percentage quantity of saline ions in the blood fed along said second tube (8) comprise a syringe by which the operator injects a predetermined quantity of saline ions into said second tube (8).

**8)** An apparatus as claimed in any one of the foregoing Claims from 1 to 6, characterized in that said means (18) for varying the percentage quantity of saline ions in the blood fed along said second tube (8) comprise a device (18) controlled by said processing means (21), for infusing a predetermined quantity of saline ions into said second tube (8).

**9)** An apparatus as claimed in Claim 8, characterized in that said device (18) provides for a single, fixed infusion of saline ions.

**10)** An apparatus as claimed in Claim 8, characterized in that said device (18) provides for a number of timed, programmed infusions of saline ions.

**11)** An apparatus as claimed in any one of the foregoing Claims from 1 to 6, characterized in that said means (18) for varying the percentage quantity of saline ions in the blood fed along said second tube (8) comprise means (21) for varying the operating characteristics of said dialysis machine (13), e.g. for varying the saline composition of the dialysis solution, for varying the supply of the dialysis solution, etc..

**12)** An apparatus as claimed in any one of the foregoing Claims, characterized in that said processing means comprise an electronic control unit (21) in turn comprising a central data processing unit (22), a memory block (23), and a timing block (24); said control unit (21) also being connecting to a data programming keyboard (25) and a programmed data and processing result display (26).

**13)** A method of determining the amount of purified blood recirculated into the external blood circuit in a dialysis apparatus comprising:
a filter assembly (2, 6);
a first tube (3) for feeding the blood to be purified from an artery (4) of the patient to said filter assembly (2, 6);
a second tube (8) for feeding the purified blood from said filter assembly (2, 6) to a vein (9) of the patient, said vein (9) communicating hydraulically with said artery (4) via a fistula (10); and
a dialysis machine (13) for preparing the dialysis solution whereby the toxic substances in the blood are removed by said filter assembly (2, 6);
characterized in that it comprises:
a first step in which means (18) vary the percentage quantity of saline ions in the blood fed along said second tube (8);
a second step in which means (16) detect the percentage quantity of saline ions in the blood fed along said second tube (8) following said variation;
a third step in which means (17 or 12) detect the percentage quantity of saline ions in the blood fed along said first tube (3) following said variation; and
a fourth step in which means (21) process the above findings; the outcome of said processing indicating the amount of purified blood recirculated from said vein (9), via said fistula (10), to said artery (4), and therefore indicating the effectiveness of the dialysis treatment.

**14)** A method as claimed in Claim 13, characterized in that it comprises a step, prior to said first step, wherein a number of values are detected along said first tube (3) and along said second tube (8) until the detected values differ from corresponding previously detected values by a quantity below a corresponding predetermined threshold value.

**15)** A method as claimed in Claim 13 and/or 14, characterized in that, in said second and third steps, a number of values are detected within a predetermined time, until a respective peak value is attained; said peak values being taken as those corresponding to the percentage quantity of saline ions in the blood fed along said first tube (3), and to the percentage quantity of saline ions in the blood fed along said second tube (8) following the set variation in the quantity of saline ions in the blood fed along said second tube (8).
